# FASCICULE DE BREVET EUROPEEN

(11) **EP 2 170 448 B1**
(45) Date de publication et mention de la délivrance du brevet: **18.01.2017**
(21) Numéro de dépôt: 08774649.1
(22) Date de dépôt: 02.07.2008
(51) Int. Cl.: A61M 25/00, A61M 39/06

(54) **DISPOSITIF D'INTRODUCTION D'UN FIL DE GUIDAGE DE CATHÉTER DANS UN VAISSEAU**
VORRICHTUNG ZUR EINFÜHRUNG EINES KATHETERFÜHRUNGSDRAHTS IN EIN GEFÄSS
DEVICE FOR INTRODUCING A CATHETER GUIDE WIRE INTO A VESSEL

(30) Priorité: 02.07.2007 FR 0756222
(43) Date de publication de la demande: 07.04.2010
(73) Titulaire: Vygon, 95440 Ecouen (FR)
(72) Inventeur: CARREZ, Jean-Luc, 95440 Ecouen (FR); DALLE, Valery, 60270 Gouvieux (FR); GUYOMARC'H, Pierrick, 95120 Ermont (FR)
(74) Mandataire: Regimbeau
(86) Numéro de dépôt international: PCT/EP2008/058515
(87) Numéro de publication internationale: WO 2009/004031

(56) Documents cités:
- WO-A-99/12600
- US-A- 5 125 905
- US-A1- 2004 122 416
- US-A1- 2005 177 043

## Description

L'invention se situe dans le domaine de l'instrumentation médicale, plus précisément des cathéters destinés à être introduits dans la circulation sanguine ou lymphatique des patients.

La présente invention concerne un dispositif permettant l'introduction d'un fil de guidage souple, à extrémité en J, à l'intérieur de l'appareil circulatoire d'un patient, ce fil de guidage permettant ultérieurement la mise en place d'un cathéter.

Les cathéters veineux centraux peuvent être introduits dans les veines par coulissement autour d'un guide spiralé et souple, ce dernier y ayant été préalablement introduit au moyen d'une aiguille.

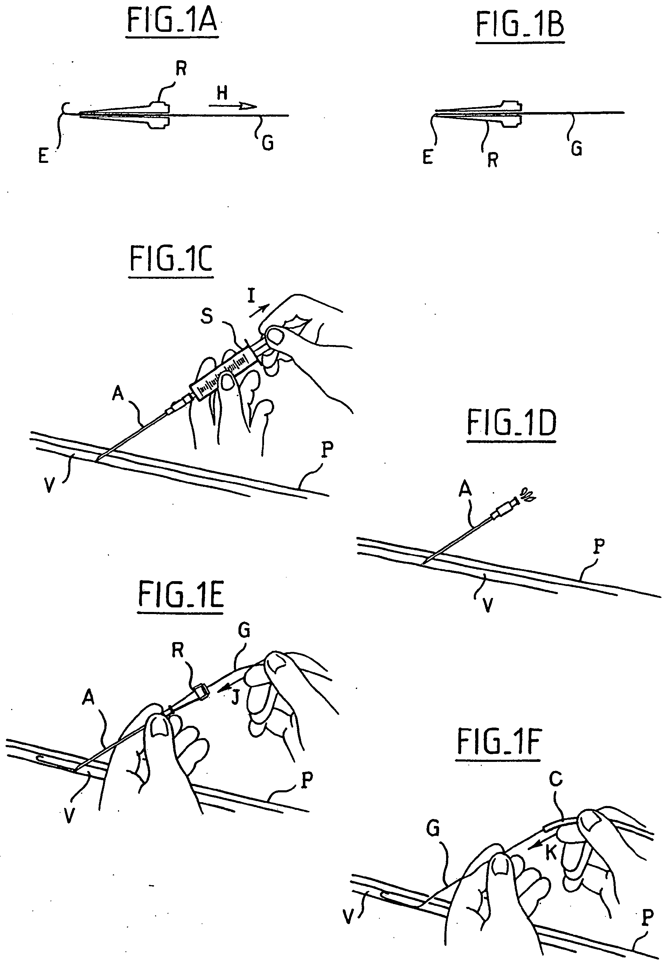

Cette technique est connue de l'homme du métier sous l'appellation de "technique de Seldinger". Elle va maintenant être décrite brièvement en faisant référence aux figures 1A à 1F jointes qui sont des schémas en représentant les principales étapes.

En ce reportant à la figure 1A, on peut voir que lorsqu'un praticien veut insérer un cathéter dans une veine dont les parois sont assez fines, il utilise pour ce faire un guide spiralé souple G dont l'extrémité distale E présente une forme recourbée sur elle-même, dite "en J". La forme spécifique de cette extrémité distale E permet son introduction à l'intérieur de la veine, sans endommager les parois de celle-ci, ni les transpercer.

Toutefois, il est nécessaire de redresser l'extrémité distale en J de ce fil de guidage avant de l'introduire dans la veine. A cette fin, le praticien utilise un redresseur R de forme conique, muni d'une lumière axiale de faible diamètre. Il introduit le fil de guidage G par l'extrémité distale E du redresseur R et exerce une traction sur celui-ci (flèche H), jusqu'à redresser l'extrémité distale E de ce fil. Il se trouve alors dans la situation représentée sur la figure 1B.

Ensuite, comme on peut le voir sur la figure 1C, le praticien utilise une seringue S munie d'une aiguille de ponction A pour traverser la peau P du patient. Une fois que le biseau de l'aiguille A se trouve dans les chairs et sous la peau, il tire sur le piston de la seringue (flèche I), en créant ainsi un vide dans celle-ci et dans l'aiguille. Le praticien pousse ensuite sur l'aiguille et au moment où le biseau de celle-ci pénètre dans la veine V, le sang est aspiré à l'intérieur de la seringue S, de sorte qu'il peut constater visuellement que le biseau de l'aiguille est bien positionné dans la veine V.

Le praticien retire alors la seringue S de l'embase de l'aiguille A, cette situation étant représentée sur la figure 1D. Puis, il introduit à l'intérieur de l'embase de l'aiguille l'extrémité distale du redresseur R, à l'intérieur duquel se trouve le fil de guidage G en position redressée.

Le praticien pousse alors le fil de guidage G au travers du redresseur R et de l'aiguille A, jusque dans la veine V (flèche J). Cette situation est représentée sur la figure 1E.

La faible distance entre l'extrémité distale du redresseur R et l'extrémité proximale de l'embase de l'aiguille A ne permet pas à l'extrémité en J du fil de guidage de se recourber, de sorte que celui-ci est obligé d'avancer en ligne droite jusque dans la veine V.

Enfin, le praticien enlève le redresseur R et l'aiguille A en les faisant coulisser le long du fil de guidage G puis il engage le tube du cathéter C sur le fil de guidage G (flèche K), jusqu'à ce que le cathéter s'engage sur une certaine distance dans la veine V, comme représenté sur la figure 1F. Il est aidé en cela par des marques présentes sur le tube du cathéter qui lui permettent de se repérer.

Cette technique présente toutefois des inconvénients en ce qu'elle nécessite de nombreuses manipulations et en ce que les pertes de sang peuvent être importantes, notamment lorsqu'on déconnecte la seringue S de l'aiguille A, comme cela est représenté sur la figure 1D.

Par ailleurs, lorsque l'on souhaite insérer un cathéter à l'intérieur d'une artère, on notera que le fil de guidage G ne présente plus nécessairement une extrémité en J, car les parois des artères étant plus résistantes et plus dures, le risque de les transpercer est moindre.

La technique d'introduction du cathéter est la même que celle qui vient d'être décrite pour une veine, si ce n'est qu'il n'est plus nécessaire d'utiliser une seringue pour visualiser le reflux sanguin, et donc la bonne position du biseau de l'aiguille dans l'artère. Toutefois, compte tenu de la pression régnant à l'intérieur des artères, les pertes de sang peuvent être beaucoup plus importantes avant l'introduction complète du fil de guidage G à l'intérieur de l'artère.

Afin d'améliorer la technique de Seldinger décrite précédemment, et également de limiter les pertes de sang, plusieurs dispositifs ont été mis au point. Deux d'entre eux sont décrits dans les documents WO 90/11098 et WO 99/12600.

Ces dispositifs consistent respectivement en une seringue dont le piston a été modifié afin de permettre l'introduction d'un cathéter sans pertes de sang et en une seringue munie d'un interrupteur.

Toutefois, ces dispositifs sont complexes, ce qui les rend plus coûteux à fabriquer et plus compliqués à utiliser. En effet, lors d'une utilisation de l'un de ces deux systèmes, le praticien se trouve à ponctionner avec un équipement encombrant, l'aiguille plus la seringue plus le redresseur de guide plus le guide enroulé dans un tube protecteur. D'autre part, les guides sont souvent longs (deux fois la longueur du cathéter), et, afin d'éviter qu'ils trainent sur le champ avant introduction (= faute d'aseptie), ils sont dans un tube protecteur long et encombrant.

On connaît également d'après le document US-5 336 192, un raccord, apte à être monté sur une aiguille de ponction ou entre cette aiguille et une seringue, pour l'introduction d'un cathéter d'angiographie à l'intérieur d'un vaisseau sanguin ou lymphatique.

Ce raccord comporte intérieurement un disque en élastomère fendu. Lorsque le raccord est connecté à une seringue, le cône de celle-ci écarte les parois du disque, ce qui permet la communication entre la seringue et l'aiguille, pour les opérations de vide et d'aspiration. Après la déconnexion de la seringue, le disque se referme en empêchant toute perte de sang.

Ce document décrit également la possibilité d'utiliser un redresseur de fil de guidage et d'introduire celui-ci dans le raccord en amont du disque en élastomère, puis de pousser le fil de guidage au travers de la fente de ce disque jusque dans la veine.

Toutefois, un tel dispositif présente l'inconvénient de ne pas être parfaitement étanche, car il est difficile de garantir l'étanchéité parfaite d'une fente autour d'un fil de guidage de forme cylindrique.

L'invention a pour but de remédier à ces inconvénients

A cet effet, l'invention concerne un dispositif pour l'introduction d'un fil à extrémité en J, dans un vaisseau sanguin ou lymphatique d'un patient, ce fil servant au guidage d'un cathéter, ce dispositif comprenant un connecteur et un redresseur de l'extrémité en J dudit fil de guidage, ledit connecteur comprenant un corps traversé par un alésage axial qui s'étend entre ses deux extrémités opposées dites "distale" et "proximale", lesdites extrémités distale et proximale permettant une liaison de type Luer respectivement avec l'embase d'une aiguille et avec l'extrémité distale d'une seringue.

Conformément à l'invention, ledit connecteur supporte intérieurement une valve d'étanchéité munie d'une fente coupant l'axe dudit alésage axial et ledit alésage axial comprend dans sa portion située entre ladite valve d'étanchéité et l'extrémité distale du connecteur, une surface, dite "d'appariement étanche" dont la forme est telle qu'elle permet un appariement étanche aux liquides avec la surface extérieure de l'extrémité distale dudit redresseur, lorsque ce dernier est introduit dans ledit connecteur, au travers de ladite valve d'étanchéité.

Selon d'autres caractéristiques avantageuses et non limitatives de l'invention, prises seules ou en combinaison :
- ladite surface d'appariement étanche est de forme tronconique, sa conicité diminuant en direction de l'extrémité distale du connecteur ;
- l'extrémité distale dudit connecteur est de forme tubulaire et sa longueur est telle qu'elle peut être enfoncée à l'intérieur de l'embase d'une aiguille, suffisamment profondément pour que l'extrémité en J dudit fil de guidage ne puisse se recourber sur elle-même, lorsque ce fil est inséré dans l'aiguille au travers dudit connecteur ;
- l'extrémité proximale dudit connecteur est un orifice dont le diamètre est adapté au diamètre extérieur d'une portion de la surface extérieure dudit redresseur, de façon à garantir une liaison étanche aux liquides avec celui-ci lorsque ledit redresseur est introduit dans ledit connecteur au travers de la valve d'étanchéité ;
- ledit connecteur est en deux parties, dites respectivement "coiffe" et "verrou", ladite coiffe incluant l'extrémité proximale du connecteur et le verrou son extrémité distale, la coiffe et le verrou étant munis de moyens d'assemblage complémentaires et maintenant ladite valve d'étanchéité lorsqu'ils sont assemblés ;
- ladite valve d'étanchéité a une forme en bec de canard ;
- ledit redresseur est percé d'une lumière axiale dont le diamètre est suffisamment grand pour laisser passer le fil de guidage lorsqu'on son extrémité en J est déployée et suffisamment petit pour éviter que cette extrémité en J ne se recourbe sur elle-même et la surface extérieure de l'extrémité distale de ce redresseur présente une forme et des dimensions appropriées pour assurer un appariement étanche aux liquides avec ladite surface d'appariement étanche du connecteur.

D'autres caractéristiques et avantages de l'invention apparaîtront de la description qui va maintenant en être faite, en référence aux dessins annexés, qui en représentent, à titre indicatif mais non limitatif, un mode de réalisation possible.

Sur ces dessins :
- les figures 1A à 1F sont des schémas représentant les différentes phases successives d'introduction d'un fil de guidage, puis d'un cathéter, à l'intérieur d'un vaisseau d'un patient selon la technique dite "de Seldinger",
- les figures 2 et 3 sont des vues respectivement en coupe longitudinale et en perspective éclatée de la partie connecteur du dispositif conforme à l'invention, la figure 3 étant à une échelle inférieure, à celle de la figure 2,
- la figure 4 est une vue en perspective du dispositif conforme à l'invention monté sur une aiguille,
- la figure 5 est une vue en coupe longitudinale axiale du connecteur, ce dernier étant monté entre l'extrémité d'une seringue et l'embase d'une aiguille,
- et la figure 6 est une vue en coupe longitudinale axiale de ce même connecteur, la seringue ayant été remplacée par le redresseur du fil de guidage.

Le dispositif conforme à l'invention est un couple constitué d'un redresseur 1 et un connecteur 3.

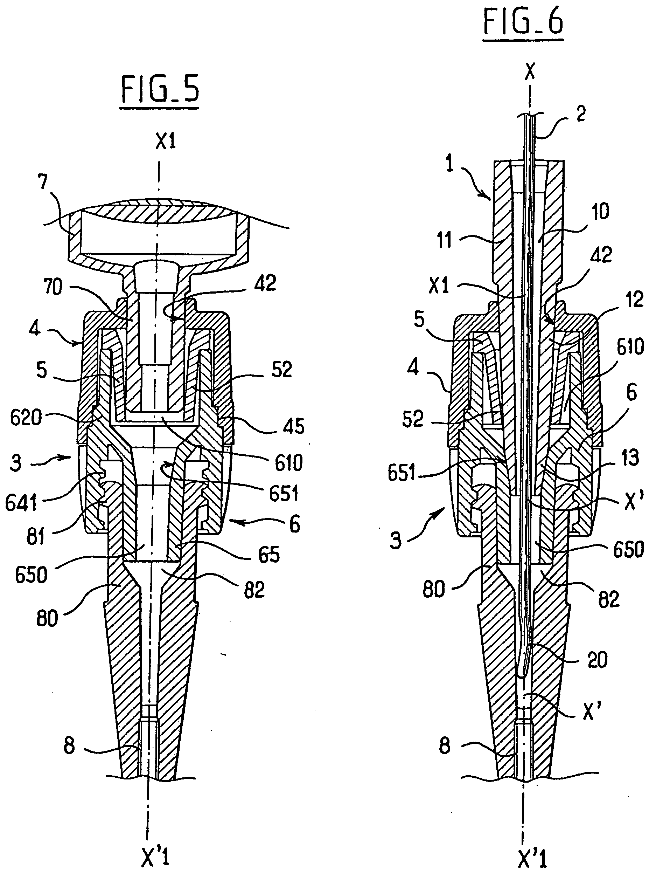

En se reportant à la figure 6, on peut voir que le redresseur 1 présente une forme générale allongée et tubulaire.

Il est traversé de part en part par une lumière 10 qui s'étend selon son axe longitudinal **X-X'**.

Le redresseur comporte trois parties, à savoir une extrémité proximale 11 de plus grand diamètre permettant la préhension par le praticien, une extrémité opposée distale 13 conique et une partie médiane 12, dont la conicité est d'une pente moindre que celle de l'extrémité distale 13 et qui s'étend entre lesdites extrémités 10 et 13.

La lumière 10 est également de forme légèrement conique, son diamètre diminuant entre l'extrémité proximale 11 et l'extrémité distale 13, afin de faciliter l'introduction d'un fil de guidage 2, un tel fil étant couramment disponible dans le commerce.

Ce fil de guidage 2 est un fil de matériau souple dont l'une des extrémités 20 est, à l'état naturel, recourbée sur elle-même d'où son nom d'extrémité en J.

L'introduction du fil 2 à l'intérieur du redresseur 1 et son positionnement de façon à en redresser l'extrémité en J 20 avant l'introduction dans un vaisseau sont identiques à ce qui a été décrit précédemment dans l'introduction de la présente demande.

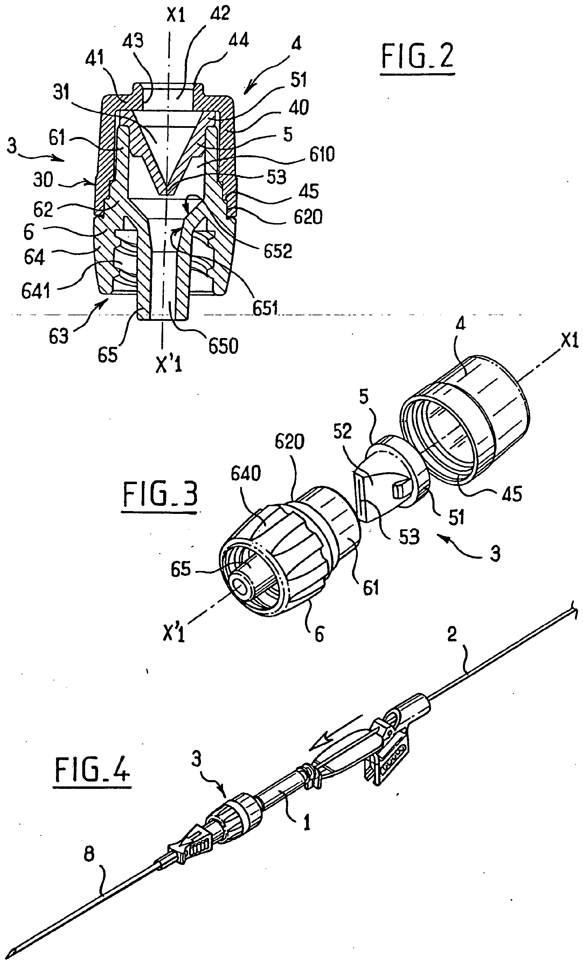

En se reportant à la figure 2, on peut voir que le connecteur 3 comprend un corps 30 de forme générale tubulaire cylindrique, traversé par un alésage axial 31 qui s'étend selon un axe longitudinal **X1-X'1.**

Le connecteur 3 est réalisé de préférence en deux parties, à savoir une coiffe 4 et un verrou 6, qui enserrent une valve d'étanchéité 5.

Ces divers éléments vont maintenant être décrits plus en détail.

La coiffe 4 présente la forme d'un manchon tubulaire d'axe longitudinal **X1-X'1** dont l'une des extrémités est repliée, de façon à définir une collerette annulaire 41 qui s'étend radialement en direction de l'intérieur du manchon 40.

Cette collerette 41 délimite ainsi un orifice 42 dont le diamètre est inférieur à celui du manchon 40 du connecteur.

Cet orifice 42 s'étend sur une faible hauteur. Sa paroi intérieure circulaire porte la référence numérique 43. Cet orifice 42 présente à son embouchure un évasement conique ou fraisure 44 qui guide et facilite l'introduction d'une seringue ou du redresseur, comme cela sera décrit ultérieurement.

A son extrémité opposée, la coiffe 4 présente sur sa face intérieure des moyens d'accouplement complémentaires 45 avec le verrou 6. Ces moyens sont par exemple un filetage, ou une soudure ultrasons, ou un collage, ou un emmanchement avec clipsage.

L'orifice 42 définit l'extrémité proximale femelle du connecteur 3 et autorise une connexion de type Luer avec une seringue standard ou le redresseur précité.

Le verrou 6 présente une forme générale tubulaire d'axe longitudinal **X1-X'1.** Il comporte une extrémité proximale cylindrique 61 dont le diamètre extérieur est très légèrement inférieur au diamètre intérieur du manchon 40, de façon à pouvoir s'engager dans celui-ci.

Le verrou 6 se poursuit par une zone médiane 62 dont la surface extérieure porte des moyens d'accouplement 620, complémentaires des moyens 45.

Enfin, l'extrémité distale 64 du verrou 6 comprend deux tubes coaxiaux 64 et 65, d'axe **X1-X'1.**

Le tube extérieur 64 comporte une surface extérieure crantée 640 qui définit une molette facilitant la préhension et la manipulation du verrou 6.

Le tube 64 présente en outre un filetage intérieur femelle 641 qui autorise le vissage étanche de l'embase d'une aiguille du type celles que l'on trouve couramment dans le commerce, notamment une aiguille de ponction.

Le tube intérieur 65 ou "fût" fait partie intégrante du reste du verrou 6. Il comprend un alésage central 650 dont la partie supérieure est évasée en forme de double tronc de cône portant les références numériques 651 et 652. La portion 652 de l'alésage, plus évasée que la portion 651, se raccorde à l'alésage 610 de plus grand diamètre ménagé à l'intérieur, de l'extrémité tubulaire 61.

La pente de la portion tronconique 651 de l'alésage est conçue pour correspondre à celle de l'extrémité distale conique 13 du redresseur 1 et garantir un appariement étanche aux liquides avec celle-ci, lorsque le redresseur 1 est introduit dans le connecteur (voir figure 6). Cette portion 651 pourrait également avoir une autre forme dès lors qu'elle garantit l'étanchéité.

En se reportant à la figure 5, on peut voir que le diamètre extérieur du fût 65 est très légèrement inférieur à celui du diamètre intérieur de l'alésage 82 ménagé dans l'embase 80 de l'aiguille 8, de façon à pouvoir être introduit dans celui-ci. L'extrémité proximale de l'embase 80 est munie, sur sa face extérieure, d'un filetage mâle 81 apte à coopérer avec le filetage femelle intérieur 641 du tube 64.

La complémentarité des filetages 81 et 641 d'une part et la complémentarité de forme du fût 65 et de l'alésage 82 d'autre part permettent la fixation de l'aiguille 8 sur le connecteur 3, par une connexion connue de l'homme du métier sous l'appellation "Luer à verrou".

Par ailleurs, comme cela apparaît mieux sur la figure 6, on notera que les dimensions, la forme et la longueur du fût 65 du connecteur sont telles qu'il pénètre jusqu'au fond de l'alésage 82 de l'embase de l'aiguille 8, de sorte que l'extrémité 20 en J du fil de guidage 2 ne peut se replier sur elle-même, lorsqu'elle pénètre dans l'embase 80 de l'aiguille 8.

La coiffe 4 et le verrou 6 sont avantageusement réalisés en matière plastique, par exemple : ABS (acrylonitrile butadiène styrène), polypropylène, POM (polyoxyméthylène), polycarbonate, polyamide.

La valve 5 est de préférence une valve anti-retour, en forme de bec de canard, réalisée en matière plastique résiliante, par exemple en élastomère, ou encore du silicone, du caoutchouc synthétique comme le polyisoprène, ou des thermoplastiques élastomères comme du SEBS (Styrène-Ethylène-Butylène-Styrène).

Comme cela apparaît mieux sur la figure 3, elle comprend une extrémité proximale annulaire 51 et une extrémité distale 52 en forme de biseau. Cette extrémité distale est percée d'une fente 53 qui coupe l'axe **X1-X'1.**

Comme on peut le voir sur la figure 2, lorsque la coiffe 4 et le verrou 6 sont assemblés, ils viennent enserrer l'extrémité proximale 51 de la valve 5 entre la collerette 41 et le rebord supérieur du manchon 61, de façon à maintenir la valve d'étanchéité 5 à l'intérieur du connecteur.

L'utilisation du connecteur 3 et du redresseur 1 conforme à l'invention vont maintenant être décrits.

Le praticien introduit l'extrémité distale 70 ou cône de la seringue 7 à l'intérieur de l'extrémité proximale 42 du connecteur 3, de sorte que cette extrémité 70 pénètre à l'intérieur de la valve 5 en bec de canard et repousse latéralement les parois de celle-ci.

Cette position est illustrée sur la figure 5.

Le caractère résiliant du matériau élastomère constituant ladite valve 5 tend à plaquer les parois biseautées 52 de celle-ci contre l'extrémité distale 70, de la seringue 7.

De plus, l'orifice 42 coopère avec l'extrémité distale 70, de façon à garantir entre ces deux éléments une liaison étanche. Le diamètre de l'orifice 42 est dimensionné en conséquence.

Le praticien visse également l'embase 80 de l'aiguille 8 autour du fût 65 du connecteur.

Comme on peut le voir sur la figure 5, la longueur courte de l'entrée 42 de la coiffe 4 permet au cône 70 de la seringue de rentrer sur une longueur importante à l'intérieur du connecteur, ce qui permet ensuite une ouverture également importante de la valve en bec de canard 5.

Le praticien enfonce la seringue sous la peau et tire sur le piston de la seringue, en créant ainsi un vide dans celle-ci et dans l'aiguille 8. Ce vide peut être obtenu grâce à l'étanchéité existant entre l'orifice 42 du capot 4 et la seringue.

Lorsque le praticien pousse l'aiguille dans la veine, puis retire la seringue, la valve 5 reprend sa position fermée illustrée à la figure 2 et garantit l'étanchéité aux liquides du dispositif. Le sang ne peut donc s'échapper hors du connecteur, comme cela était le cas dans les dispositifs de l'art antérieur (voir figure 1D).

Le praticien positionne ensuite le fil de guidage 2 à l'intérieur du redresseur 1, de façon à en redresser l'extrémité distale 20 en J, comme expliqué précédemment.

Il introduit ensuite le redresseur 1 coaxialement à l'intérieur du connecteur 3, de sorte que le redresseur 1 repousse les parois de la valve 5 vers l'extérieur et est guidé jusqu'à ce que son extrémité distale 13 vienne en butée contre la face intérieure tronconique 651 du verrou 6, en créant à ce niveau une liaison étanche.

Cette surface 651 constitue donc une "surface d'appariement" étanche aux liquides.

Par ailleurs, la liaison étanche est également assurée entre la portion médiane 12 du redresseur 1 et les parois de l'entrée proximale 42 du connecteur.

Contrairement au dispositif connu de l'état de la technique, c'est le redresseur 1 qui traverse la valve 5 et l'étanchéité aux liquides est assurée en aval, au niveau de la surface 651. En aucun cas, le fil de guidage 2 n'est en contact avec la valve 5. Un éventuel reflux de sang est ainsi empêché. Ainsi, le fil de guidage 2 est apte à coulisser librement dans un canal tubulaire ainsi formé, et le praticien n'a pas à redouter un blocage quelconque du fil de guidage qui pourrait conduire à un endommagement de la veine.

Par ailleurs, le sang peut difficilement passer entre le fil de guidage 2 et l'alésage interne 10 du redresseur 1, et ce, d'autant plus que le fil de guidage 2 est poussé en direction distale. Par ailleurs, le sang présent dans le connecteur sous la valve 5 ne peut pas sortir par l'entrée de la coiffe, car le redresseur 1 obture de façon étanche la cavité 42. La pression exercée par les parois biseautées 52 de la valve 5 contribue également à garantir l'étanchéité.

Le praticien pousse le fil de guidage 2 jusqu'à que celui-ci pénètre dans la veine (voir la figure 4), puis retire l'ensemble aiguille 8, connecteur 3 et redresseur 1. Il peut alors faire coulisser le cathéter le long du fil de guidage 2, comme décrit précédemment en liaison avec la figure 1F.

Le dispositif conforme à l'invention est simple à utiliser et permet d'éviter les pertes de sang que l'on peut observer avec les dispositifs de l'art antérieur.

## Revendications

1. Dispositif pour l'introduction d'un fil (2) à extrémité en J (20), dans un vaisseau sanguin ou lymphatique d'un patient, ce fil servant au guidage d'un cathéter, ce dispositif comprenant un connecteur (3, 4, 6) et un redresseur (1) de l'extrémité en J dudit fil de guidage, ledit connecteur (3, 4, 6) comprenant un corps (30) traversé par un alésage axial (31, 610, 650) qui s'étend entre ses deux extrémités opposées dites "distale" (65) et "proximale" (42), lesdites extrémités distale (65) et proximale (42) permettant une liaison de type Luer respectivement avec une embase (80) d'une aiguille (8) et avec une extrémité distale (70) d'une seringue (7), **caractérisé en ce que** ledit connecteur supporte intérieurement une valve d'étanchéité (5) munie d'une fente (53) coupant un axe **(X-X'1)** dudit alésage axial (31, 610, 650) et **en ce que** ledit alésage axial (31, 610, 650) comprend dans une portion située entre ladite valve d'étanchéité et l'extrémité distale (65) du connecteur, une surface (651), dite "d'appariement étanche" dont la forme est telle qu'elle permet un appariement étanche aux liquides avec une surface extérieure de l'extrémité distale (13) dudit redresseur (1), lorsque ce dernier est introduit dans ledit connecteur (3, 4, 6), au travers de ladite valve d'étanchéité (5).

2. Dispositif selon la revendication 1, **caractérisé en ce que** ladite surface d'appariement étanche (651) est de forme tronconique, sa conicité diminuant en direction de l'extrémité distale (65) du connecteur (3, 4, 6).

3. Dispositif selon la revendication 1 ou 2, **caractérisé en ce que** l'extrémité distale (65) dudit connecteur (3, 4, 6) est de forme tubulaire et **en ce que** sa longueur est telle qu'elle peut être enfoncée à l'intérieur de l'embase (80) d'une aiguille (8), suffisamment profondément pour que l'extrémité en J dudit fil de guidage ne puisse se recourber sur elle-même, lorsque ce fil est inséré dans l'aiguille (8) au travers dudit connecteur (3, 4, 6).

4. Dispositif selon l'une des revendications précédentes, **caractérisé en ce que** l'extrémité proximale (42) dudit connecteur (3, 4, 6) est un orifice dont le diamètre est adapté au diamètre extérieur d'une portion (12) de la surface extérieure dudit redresseur (1), de façon à garantir une liaison étanche aux liquides avec celui-ci lorsque ledit redresseur (1) est introduit dans ledit connecteur (3, 4, 6) au travers de la valve d'étanchéité (5).

5. Dispositif selon l'une des revendications précédentes, **caractérisé en ce que** ledit connecteur (3, 4, 6) est en deux parties, dites respectivement "coiffe" (4) et "verrou" (6), ladite coiffe (4) incluant l'extrémité proximale (42) du connecteur et le verrou (6) son extrémité distale (65), la coiffe (4) et le verrou (6) étant munis de moyens d'assemblage complémentaires (45, 620) et maintenant ladite valve d'étanchéité (5) lorsqu'ils sont assemblés.

6. Dispositif selon l'une des revendications précédentes, **caractérisé en ce que** ladite valve d'étanchéité (5) a une forme en bec de canard.

7. Dispositif selon l'une des revendications précédentes, **caractérisé en ce que** ledit redresseur (1) est percé d'une lumière axiale (10) dont le diamètre est suffisamment grand pour laisser passer le fil de guidage lorsque son extrémité en J est déployée et suffisamment petit pour éviter que cette extrémité en J ne se recourbe sur elle-même et **en ce que** la surface extérieure de l'extrémité distale (13) de ce redresseur (1) présente une forme et des dimensions appropriées pour assurer un appariement étanche aux liquides avec ladite surface d'appariement étanche (651) du connecteur (3, 4, 6).

## Patentansprüche

1. Vorrichtung zum Einführen eines Fadens (2) mit J-förmigem Ende (20) in ein Blut- oder Lymphgefäß eines Patienten, wobei dieser Faden als Führung eines Katheters dient, wobei diese Vorrichtung einen Konnektor (3, 4, 6) und einen Gleichrichter (1) des J-förmigen Endes des Führungsfadens umfasst, wobei der Konnektor (3, 4, 6) einen Körper (30) umfasst, der durch eine axiale Bohrung (31, 610, 650) durchquert wird, die sich zwischen ihren zwei entgegengesetzten Enden, bezeichnet als "distales" (65) und "proximales" (42) erstreckt, wobei das distale (65) und proximale (42) Ende eine Verbindung vom Typ Luer jeweils mit einer Basis (80) einer Nadel (8) und mit einem distalen Ende (70) einer Spritze (7) erlauben, **dadurch gekennzeichnet, dass** der Konnektor im Innern ein Abdichtventil (5) trägt, das mit einem Schlitz (53) versehen ist, der eine Achse (X-X'1) der axialen Bohrung (31, 610, 650) schneidet und dass die axiale Bohrung (31, 610, 650) in einem Abschnitt, der sich zwischen dem Abdichtventil und dem distalen Ende (65) des Konnektors befindet, eine Oberfläche (651) umfasst, bezeichnet als "abgedichtete Paarung", deren Form derart ist, dass sie eine gegen Flüssigkeiten abgedichtete Paarung mit einer äußeren Oberfläche und dem distalen Ende (13) des Gleichrichters (1) erlaubt, wenn letzterer durch das Abdichtventil (5) in den Konnektor (3, 4, 6) eingeführt wird.

2. Vorrichtung gemäß Anspruch 1, **dadurch gekennzeichnet, dass** die abgedichtete Paarungsoberfläche (651) eine kegelstumpfartige Form aufweist, wobei ihre Konizität in Richtung des distalen Endes (65) des Konnektors (3, 4, 6) abnimmt.

3. Vorrichtung gemäß Anspruch 1 oder 2, **dadurch gekennzeichnet, dass** das distale Ende (65) des Konnektors (3, 4, 6) röhrenförmig ist und dass seine Länge derart ist, dass sie in das Innere der Basis (80) einer Nadel (8) eingedrückt werden kann, die ausreichend tief ist, damit das J-förmige Ende des Führungsfadens sich nicht zu sich selbst zurückbiegen kann, wenn der Faden durch den Konnektor (3, 4, 6) in die Nadel (8) eingeführt wird.

4. Vorrichtung gemäß einem der voranstehenden Ansprüche, **dadurch gekennzeichnet, dass** das proximale Ende (42) des Konnektors (3, 4, 6) ein Loch ist, dessen Durchmesser an den Außendurchmesser eines Abschnitts (12) der äußeren Oberfläche des Gleichrichters (1) derart angepasst ist, dass eine gegen Flüssigkeiten abgedichtete Verbindung mit diesem garantiert ist, wenn der Gleichrichter (1) durch das Abdichtventil (5) in den Konnektor (3, 4, 6) eingeführt wird.

5. Vorrichtung gemäß einem der voranstehenden Ansprüche, **dadurch gekennzeichnet, dass** der Konnektor (3, 4, 6) sich in zwei Teilen präsentiert, jeweils bezeichnet als "Kappe" (4) und "Riegel" (6), wobei die Kappe (4) das proximale Ende (42) des Konnektors einschließt und der Riegel (6) sein distales Ende (65), wobei die Kappe (4) und der Riegel (6) mit komplementären Montagemitteln (45, 620) versehen sind und das Abdichtventil (5) gehalten wird, wenn sie montiert sind.

6. Vorrichtung gemäß einem der voranstehenden Ansprüche, **dadurch gekennzeichnet, dass** das Abdichtventil (5) die Form eines Entenschnabels aufweist.

7. Vorrichtung gemäß einem der voranstehenden Ansprüche, **dadurch gekennzeichnet, dass** der Gleichrichter (1) mit einer axialen Öffnung (10) durchbohrt ist, deren Durchmesser ausreichend groß ist, um den Führungsfaden durchtreten zu lassen, wenn sein J-förmiges Ende ausgefahren ist, und ausreichend klein ist, um zu verhindern, dass dieses J-förmige Ende sich zu sich selbst zurückbiegt und dass die externe Oberfläche des distalen Endes (13) dieses Gleichrichters (1) eine Form und Abmessungen aufweist, die geeignet sind, um eine gegen Flüssigkeiten abgedichtete Paarung mit der abgedichteten Paarungsoberfläche (651) des Konnektors (3, 4, 6) aufzuweisen.

## Claims

1. A device for introducing a J-ended (20) wire (2) into a patient's blood or lymphatic vessel, this wire being used for guiding a catheter, this device comprising a connector (3, 4, 6) and a straightener (1) of the J-end of said guiding wire, said connector (3, 4, 6) comprising a body (30) crossed by an axial bore (31, 610, 650) extending between its two opposite ends known as "distal" (65) and "proximal" (42) ends, said distal (65) and proximal (42) ends enabling a Luer-type connection respectively with a hub (80) of a needle (8) and with a distal end (70) of a syringe (7), **characterised in that** said connector internally supports a sealing valve (5) provided with a slot (53) intersecting an axis **(X-X'1)** of said axial bore (31, 610, 650) and **in that** said axial bore (31, 610, 650) comprises in a portion located between said sealing valve and the distal end (65) of the connector, a surface (651), known as a "tight matching" surface the shape of which is such that it enables a liquid-tight matching with an outer surface of the distal end (13) of said straightener (1), when the latter is introduced in said connector (3, 4, 6) through said sealing valve (5).

2. The device according to claim 1, **characterised in that** said tight matching surface (651) has a frustoconical shape, its conicity decreasing towards the distal end (65) of the connector (3 4, 6).

3. The device according to claim 1 or 2, **characterised in that** the distal end (65) of said connector (3, 4, 6) has a tubular shape and **in that** its length is such that it can be pushed inside the hub (80) of a needle (8), deep enough for the J-end of said guiding wire to be unable to bend on itself, when this wire is inserted in the needle (8) through said connector (3, 4, 6).

4. The device according to one of the preceding claims, **characterised in that** the proximal end (42) of said connector (3, 4, 6) is a hole the diameter of which is adapted to the outer diameter of a portion (12) of the outer surface of said straightener (1), so as to ensure a liquid-tight connection with the latter when said straightener (1) is introduced in said connector (3, 4, 6) through the sealing valve (5).

5. The device according to one of the preceding claims, **characterised in that** said connector (3, 4, 6) is in two parts, respectively known as a "cap" (4) and a "lock" (6), said cap (4) including the proximal end (42) of the connector and the lock (6) its distal end (65), the cap (4) and the lock (6) being provided with complementary assembly means (45, 620) and holding said sealing valve (5) when they are assembled.

6. The device according to one of the preceding claims, **characterised in that** said sealing valve (5) has a duckbill shape.

7. The device according to one of the preceding claims, **characterised in that** said straightener (1) is drilled with an axial port (10) the diameter of which is large enough to let the guiding wire through when its J-end is extended and small enough to prevent this J-end from bending on itself and **in that** the outer surface of the distal end (13) of this straightener (1) has appropriate shape and dimensions to ensure a liquid tight matching with said tight matching surface (651) of the connector (3, 4, 6).
